Europäisches Patentamt

European Patent Office

Office européen des brevets

(19)

(11) Veröffentlichungsnummer : **0 141 316**
**B1**

(12) **EUROPÄISCHE PATENTSCHRIFT**

(45) Veröffentlichungstag der Patentschrift :
22.10.86

(21) Anmeldenummer : 84112282.3

(22) Anmeldetag : 12.10.84

(51) Int. Cl.⁴ : **C 07 C 11/18, C 07 C 7/10,**
**C 07 C 7/08**

(54) Verfahren zur Gewinnung von Isopren aus einem C5-Kohlenwasserstoffgemisch.

(30) Priorität : 21.10.83 DE 3338269

(43) Veröffentlichungstag der Anmeldung :
15.05.85 Patentblatt 85/20

(45) Bekanntmachung des Hinweises auf die Patenterteilung : 22.10.86 Patentblatt 86/43

(84) Benannte Vertragsstaaten :
AT BE DE FR GB IT NL SE

(56) Entgegenhaltungen :
DE-A- 2 165 454

(73) Patentinhaber : BASF Aktiengesellschaft
Carl-Bosch-Strasse 38
D-6700 Ludwigshafen (DE)

(72) Erfinder : Lindner, Alfred, Dr.
Ringstrasse 30
D-6712 Bobenheim-Roxheim (DE)
Erfinder : Wagner, Ulrich, Dr.
Knospstrasse 7
D-6703 Limburgerhof (DE)
Erfinder : Volkamer, Klaus, Dr.
Heidelberger Ring 21
D-6710 Frankenthal (DE)
Erfinder : Rebafka, Walter, Dr.
Lessingstrasse 4
D-6945 Hirschberg (DE)

**Beschreibung**

Die vorliegende Erfindung betrifft ein Verfahren zur Gewinnung von Isopren aus einem Isopren und Pentadien-1,3 und Cyclopentadien enthaltenden $C_5$-Kohlenwasserstoffgemisch.

Es ist bekannt, z. B. aus der deutschen Auslegeschrift 18 07 675, Isopren aus einem Isopren und Cyclopentadien enthaltenden $C_5$-Kohlenwasserstoffgemisch unter Verwendung eines selektiven Lösungsmittels durch extraktive Destillation zu gewinnen. Dieses Verfahren erfordert jedoch, um ein für die Polymerisation zu Polyisopren geeignetes Isopren von hoher Reinheit zu erhalten, die Kombination von zwei extraktiven Destillationen und einer fraktionierten Destillation. Dabei ist es weiter erforderlich, um ein Isopren mit hinreichend niedrigem Cyclopentadien-Gehalt zu erhalten, die zur extraktiven Destillation zugehörige Lösungsmittelwiedergewinnungszone (Ausgaser) unter vermindertem Druck zu betreiben. Dieses Verfahren ist daher sehr apparateaufwendig und hat einen erheblichen Energieverbrauch, der zudem noch bei erhöhter Temperatur anfällt. Ein weiterer Nachteil des bekannten Verfahrens besteht darin, daß das bei der Ausgasung in der Lösungsmittelwiedergewinnungszone in erheblichen Mengen anfallende Pentadien-1,3, Cyclopentadien und Dicyclopentadien enthaltende Diengemisch nur geringen Wert hat.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung von Isopren aus einem Isopren, Pentadien-1,3 und Cyclopentadien enthaltenden $C_5$-Kohlenwasserstoffgemisch durch Trennung des $C_5$-Kohlenwasserstoffgemischs durch Flüssig-Flüssig-Extraktion oder extraktive Destillation mit Hilfe eines selektiven Lösungsmittels in Kombination mit einer zur Flüssig-Flüssig-Extraktion oder extraktiven Destillation vorgeschalteten oder nachgeschalteten Destillation, welches dadurch gekennzeichnet ist, daß man in der vorgeschalteten oder nachgeschalteten Destillation einen Pentadien-1,3 und Cyclopentadien enthaltenden Strom abtrennt, diesen Strom katalytisch hydriert und den hydrierten Strom dem Oberteil der Flüssig-Flüssig-Extraktion oder extraktiven Destillation zuführt.

Nach dem neuen Verfahren ist es möglich, in überraschend einfacher Weise die Verfahrenstemperatur niedrig zu halten, da auf eine Ausgasung von mit Pentadien-1,3 und Cyclopentadien und aus Cyclopentadien gebildetem Dicyclopentadien beladenem Lösungsmittel, die eine erhöhte Verfahrenstemperatur erforderlich macht, ganz oder teilweise verzichtet werden kann. Die durch die Hydrierung von Pentadien-1,3 und Cyclopentadien nach dem neuen Verfahren entstehenden Hydrierprodukte wie Pentan, Pentene, Cyclopentan, Cyclopenten lassen sich in einfacher Weise im Destillat der Flüssig-Flüssig-Extraktion bzw. extraktiven Destillation als Wertprodukte abtrennen. Die bei dem bekannten Verfahren aufgrund der möglichen Dimerisierung des Cyclopentadiens zum Dicyclopentadien und ebenso aufgrund der möglichen Zersetzung von Dicyclopentadien unter Rückbildung von Cyclopentadien auftretenden Schwierigkeiten können beim erfindungsgemäßen Verfahren vermieden werden.

Die erfindungsgemäß zu Gewinnung von Isopren einzusetzenden Isopren, Pentadien-1,3 und Cyclopentadien enthaltenden $C_5$-Kohlenwasserstoffgemische werden z. B. bei der Herstellung von Ethylen und/oder Propylen durch thermisches Spalten in Gegenwart von Wasserdampf einer Petroleumfraktion, z. B. von verflüssigtem Petroleumgas (LPG), Leichtbenzin (Naphtha), Gasöl oder dergleichen als Kohlenwasserstoffreaktion erhalten. Weiterhin werden solche $C_5$-Fraktionen bei der katalytischen Dehydrierung von Pentanen und/oder Pentenen erhalten. Das $C_5$-Kohlenwasserstoffgemisch enthält in der Regel verschiedene Arten von $C_5$-Kohlenwasserstoffen, die verschiedene Grade an Ungesättigtheit aufweisen und auch noch geringe Mengen verschiedener Kohlenwasserstoffe mit C-Zahlen kleiner und/oder größer $C_5$ enthalten können. In der Regel sind in den $C_5$-Kohlenwasserstoffgemischen z. B. n-Pentan, Isopentan, Penten-1, 2-Methylbuten-1, 3-Methylbuten-1, 2-Methylbuten-2, trans-Penten-2, cis-Penten-2, Isopren, trans-Pentadien-1,3, cis-Pentadien-1,3, Pentadien-1,4, Pentin-1, Pentin-2, Isopropenylacetylen, Isopropylacetylen, Cyclopentan, Cyclopenten, Cyclopentadien enthalten. Der Gehalt der $C_5$-Kohlenwasserstoffgemische an Pentadien-1,3 liegt in der Regel im Bereich von 1 bis 25 Gew.%, vorzugsweise 2 bis 20 Gew.%, insbesondere 5 bis 15 Gew.%. Er kann jedoch auch höher oder niedriger liegen. Das Cyclopentadien kann als solches oder in dimerisierter Form als Dicyclopentadien, mit dem es in einem thermischen Gleichgewicht steht, vorliegen. Der Gehalt des Ausgangs-$C_5$-Kohlenwasserstoffgemischs an Cyclopentadien und/oder Dicyclopentadien beträgt im allgemeinen 1 bis 35 Gew.%, vorzugsweise 2 bis 30 Gew.%, insbesondere 5 bis 25 Gew.%. Er kann jedoch auch höher oder niedriger liegen.

Als selektives Lösungsmittel kommen beispielsweise Carbonsäureamide, wie Dimethylformamid, Diethylformamid, Dimethylacetamid, N-Formylmorpholin, Acetonitril, Furfurol, N-Methylpyrrolidon, Butyrolacton, Aceton und ihre Mischungen mit Wasser in Betracht. Mit besonderem Vorteil wird N-Methylpyrrolidon als selektives Lösungsmittel verwendet.

Die Trennung der $C_5$-Kohlenwasserstoffe mit Hilfe des selektiven Lösungsmittels wird in einer Flüssig-Flüssig-Extraktion oder einer extraktiven Destillation durchgeführt, wobei es vorteilhaft sein kann, die extraktive Destillation anzuwenden.

Der Pentadien-1,3 und Cyclopentadien (gegebenenfalls ganz oder teilweise als Dicyclopentadien vorliegend) enthaltende Strom, der erfindungsgemäß der katalytischen Hydrierung zu unterwerfen ist, weist im allgemeinen einen Gehalt an Pentadien-1,3 von 10 bis 95 Gew.%, vorzugsweise 20 bis 90 Gew.%, insbesondere 30 bis 80 Gew.% und einen Gehalt an Cyclopentadien von 1 bis 90 Gew.%, vorzugsweise 20 bis 80 Gew.%, insbesondere 50 bis 70 Gew.%, bezogen auf den $C_5$-Kohlenwasserstoffanteil in diesem Strom, auf. Für die Hydrierung wird im allgemeinen der üblicherweise für eine Hydrierreaktion zu verwendende Wasserstoff, beispielsweise technisch reiner Wasserstoff, eingesetzt. Der Wasserstoff kann unverdünnt oder nach Verdünnung mit einem inerten Gas, wie Stickstoff, verwendet werden. Vorzugsweise setzt man den Wasserstoff ohne Inertgaszusatz ein. Falls der Hydrierwasserstoff mit einem inerten Gas verdünnt wird, so beträgt im allgemeinen das Volumenverhältnis von Inertgas zu Hydrierwasserstoff 1 : 10 000 bis 4 : 1, vorzugsweise 1 : 1 000 bis 2,5 : 1.

Man kann die Hydrierung in der Gasphase oder in flüssiger Phase durchführen. Vorzugsweise wird die Hydrierung in flüssiger Phase durchgeführt. Man kann den zu hydrierenden, Pentadien-1,3 und Cyclopentadien enthaltenden Strom als solchen der Hydrierung unterwerfen. Es kann jedoch auch vorteilhaft sein, einen zu hydrierenden Strom zu verwenden, der inertes Lösungsmittel enthält, wobei vorzugsweise das für die Flüssig-Flüssig-Extraktion oder extraktive Destillation zu verwendende selektive Lösungsmittel als inertes Lösungsmittel verwendet wird. Mit besonderem Vorteil wird das aus dem Ausgasungsteil der Flüssig-Flüssig-Extraktion oder extraktiven Destillation erhaltene selektive Lösungsmittel, das im Ausgasungsteil ganz oder teilweise von den Kohlenwasserstoffen befreit worden ist, verwendet, wobei die Verwendung von nur teilweise entgastem selektivem Lösungsmittel bevorzugt wird. Bei der Verdünnung mit einem Lösungsmittel wird das Lösungsmittel im allgemeinen in Konzentrationen von 60 bis 99, vorzugsweise 80 bis 98, insbesondere 90 bis 97 Gew.%, im Gemisch mit dem zu hydrierenden Kohlenwasserstoffstrom, angewendet.

Man kann die Hydrierung adiabatisch oder isotherm durchführen. Zweckmäßig führt man die Reaktion bei Temperaturen zwischen 20 und 180, vorzugsweise zwischen 60 und 150 °C durch. Im allgemeinen werden Drücke von 1 bis 100 bar, vorzugsweise 5 bis 50 bar angewendet. Man kann den zu hydrierenden Pentadien-1,3 und Cyclopentadien enthaltenden Strom und den Hydrierwasserstoff im Gleich- oder im Gegenstrom führen. In einer vorteilhaften Ausführungsform des Verfahrens wird die Hydrierwärme für die Trennung des $C_5$-Kohlenwasserstoffgemischs in der Flüssig-Flüssig-Extraktion und/oder extraktiven Destillation verwendet, beispielsweise indem die Hydrierwärme über einen Aufkocher der Flüssig-Flüssig-Extraktion oder extraktiven Destillation zugeführt wird.

Als Hydrierkatalysatoren werden zweckmäßig Metalle der VIII. Gruppe des Periodensystems und/oder deren Verbindungen enthaltende Katalysatoren verwendet. Geeignete Metalle der VIII. Gruppe des Periodensystems sind beispielsweise Kobalt, Nickel und vorzugsweise die Edelmetalle, wie Palladium, Platin. Insbesondere wird Palladium verwendet. Es kann vorteilhaft sein, die Katalysatoren als Trägerkatalysatoren zu verwenden. Als Trägermaterial kommen z. B. Aktivkohle, Kieselgel, Aluminiumoxid, Diatomeenerde, Calciumcarbonat oder deren Mischungen in Betracht. Im allgemeinen beträgt der Gehalt an Metall der VIII. Gruppe des Periodensystems 0,01 bis 5 Gew.%, vorzugsweise 0,1 bis 2 Gew.%, bezogen auf den Trägerkatalysator. Man kann die ein Metall der VIII. Gruppe des Periodensystems und/oder deren Verbindungen enthaltenden Trägerkatalysatoren ohne weitere Zusätze verwenden. Es kann jedoch auch vorteilhaft sein, solche Trägerkatalysatoren zu verwenden, die zusätzlich mindestens eine Verbindung eines weiteren Metalls enthalten.

Bei der Hydrierung des Pentadien-1,3 und Cyclopentadien enthaltenden Stromes wird zweckmäßig das Pentadien-1,3 zu Pentenen und/oder Pentan und das Cyclopentadien zu Cyclopenten und/oder Cyclopentan hydriert. Der hydrierte Strom wird dem Oberteil der Flüssig-Flüssig-Extraktion oder extraktiven Destillation zugeführt. Im allgemeinen erfolgt die Zuführung des hydrierten Stromes an einem Punkt, der an oder oberhalb der Zuführung des $C_5$-Kohlenwasserstoffgemisches zur Flüssig-Flüssig-Extraktion oder extraktiven Destillation liegt, wobei eine Zuführung im oberen Drittel der Kolonne für die Flüssig-Flüssig-Extraktion bzw. extraktive Destillation bevorzugt wird. In einer bevorzugten Ausführungsform wird der hydrierte Strom zusammen mit dem selektiven Lösungsmittel dem Oberteil der Flüssig-Flüssig-Extraktion oder extraktiven Destillation zugeführt.

Eine Ausführungsform des erfindungsgemäßen Verfahrens besteht z. B. darin, daß man das Ausgangs-$C_5$-Kohlenwasserstoffgemisch zunächst in einer Destillationszone destilliert, wobei am Kopf der Destillationszone ein Isopren enthaltender Strom und am Sumpf der Destillationszone ein Pentadien-1,3 und Cyclopentadien enthaltender Strom abgezogen werden. Der Pentadien-1,3 und Cyclopentadien enthaltende Strom wird anschießend in einer Hydrierzone katalytisch hydriert, und der hydrierte Strom wird dem Oberteil einer Flüssig-Flüssig-Extraktion oder extraktiven Destillation zugeführt. Der am Kopf der vorgeschalteten Destillationszone abgezogene, Isopren enthaltende Strom wird zweckmäßig dem mittleren Teil der Flüssig-Flüssig-Extraktion oder extraktiven Destillation zugeführt, wobei sich der mittlere Teil im allgemeinen vom unteren Fünftel bis zum oberen Fünftel, vorzugsweise vom unteren Viertel bis zum oberen Viertel, insbesondere vom unteren Drittel bis zum oberen Drittel, der Zone der Flüssig-Flüssig-Extraktion oder extraktiven Destillation erstreckt. Die der Flüssig-Flüssig-Extraktion oder extraktiven Destillation im mittle-

ren Teil zugeführten $C_5$-Kohlenwasserstoffe werden in ein die im selektiven Lösungsmittel weniger als Isopren löslichen Kohlenwasserstoffe, z. B. n-Pentan, Isopentan, Penten-1,3-Methylbuten-1, 2-Methylbuten-2, Penten-2, Cyclopentan, Cyclopenten, enthaltendes Destillat, einen Strom aus Isopren und einen die im selektiven Lösungsmittel löslicheren Kohlenwasserstoffe als Isopren, z. B. 1,3-Pentadien, 1,4-Pentadien, 2-Butin, $C_5$-Acetylene, Cyclopentadien und Cyclopenten enthaltenden Strom aufgetrennt.

Eine weitere Ausführungsform des erfindungsgemäßen Verfahrens besteht beispielsweise darin, daß man das Ausgangs-$C_5$-Kohlenwasserstoffgemisch zunächst durch Flüssig-Flüssig-Extraktion oder extraktive Destillation in ein die im selektiven Lösungsmittel weniger als Isopren löslichen Kohlenwasserstoffe enthaltendes Destillat, einen Isopren, Pentadien-1,3 und Cyclopentadien enthaltenden Strom und einen die im selektiven Lösungsmittel löslicheren Kohlenwasserstoffe als Isopren enthaltenden Strom auftrennt. Dabei wird das Ausgangs-$C_5$-Kohlenwasserstoffgemisch im allgemeinen im mittleren Bereich der Flüssig-Flüssig-Extraktion oder extraktiven Destillation, bestehend aus Verstärkerteil, Abtriebsteil und Ausgasungsteil, im allgemeinen in einem Bereich, der sich vom unteren Fünftel bis zum oberen Fünftel, vorzugsweise vom unteren Viertel bis zum oberen Viertel, insbesondere vom unteren Drittel bis zum oberen Drittel der Zone der Flüssig-Flüssig-Extraktion oder extraktiven Destillation erstreckt, zugeführt. Der Isopren, Pentadien-1,3 und Cyclopentadien enthaltende Strom wird zweckmäßig an einem unterhalb der Zuführung des Ausgangs-$C_5$-Kohlenwassergemisches und oberhalb des Sumpfes liegenden Punkt, abgezogen, während der die im selektiven Lösungsmittel löslicheren Kohlenwasserstoffe enthaltende Strom zweckmäßig am Sumpf der Flüssig-Flüssig-Extraktion oder extraktiven Destillation abgezogen wird. Der Isopren, Pentadien-1,3 und Cyclopentadien enthaltende Strom, der noch dampfdruckmäßig mitgenommenes selektives Lösungsmittel enthalten kann, wird in einer Destillationszone aufgetrennt, wobei am Kopf der Destillationszone ein Strom aus Isopren und am Sumpf der Destillationszone ein Pentadien-1,3 und Cyclopentadien enthaltender Strom abgezogen werden. Die Destillationszone wird vorzugsweise als fraktionierte Destillation betrieben. Es ist jedoch auch möglich, die Destillationszone als extraktive Destillation zu betreiben. Der am Sumpf der Destillationszone abgezogene, Pentadien-1,3 und Cyclopentadien enthaltende Strom wird in einer Hydrierzone katalytisch hydriert und der hydrierte Strom wird dem Oberteil der Flüssig-Flüssig-Extraktion oder extraktiven Destillation zweckmäßig an einem Punkt oberhalb der Zuführung des Ausgangs-$C_5$-Kohlenwasserstoffgemischs, zugeführt. Es kann vorteilhaft sein, den Pentadien-1,3 und Cyclopentadien enthaltenden Sumpfabzug der Destillationszone mit dem Sumpfprodukt der Flüssig-Flüssig-Extraktion oder extraktiven Destillation zu vereinigen und die vereinigten Ströme der Hydrierzone zur katalytischen Hydrierung zuzuführen und den hydrierten Strom, wie bereits beschrieben, dem Oberteil der Flüssig-Flüssig-Extraktion oder extraktiven Destillation zuzuführen. Es ist jedoch auch möglich, den Pentadien-1,3 und Cyclopentadien enthaltenden Sumpfabzug der Destillationszone dem abgezogenen ganz oder teilweise ausgegasten selektiven Lösungsmittel zuzufügen und anschließend zur katalytischen Hydrierung zuzuführen, wobei es bevorzugt ist, den Pentadien-1,3 und Cyclopentadien enthaltenden Sumpfabzug dem nur teilweise ausgegasten selektiven Lösungsmittel zuzufügen.

Isopren ist ein wertvolles Ausgangsprodukt beispielsweise für die Herstellung von Vitaminen oder Riechstoffen sowie zur Herstellung von Kautschuk.

Das nachstehende Beispiel dient der weiteren Erläuterung der Erfindung.

Beispiel

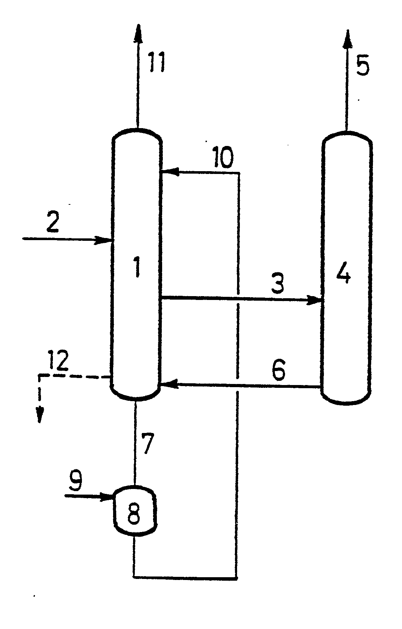

Einer (vgl. die Figur) mit wasserhaltigem N-Methylpyrrolidon (NMP) (8 Gew.% $H_2O$) als selektives Lösungsmittel betriebenen extraktiven Destillation 1 (bestehend aus Verstärkerteil, Abtriebsteil und Ausgasungsteil) wurde ein Ausgangs-$C_5$-Kohlenwasserstoffgemisch der folgenden Zusammensetzung über Leitung 2 zugeführt :

| | |
|---|---|
| Isopren | 18 Gew.% |
| Pentadien-1,3 | 10 Gew.% |
| Cyclopentadien | 16 Gew.% |
| Pentane | 30 Gew.% |
| Pentene sowie geringe Mengen von $C_4$- und $C_6$-Kohlenwasserstoffen | 26 Gew.% |

Die Sumpftemperatur in der extraktiven Destillation betrug 110 bis 130 °C. Über den Abzug 3 der extraktiven Destillation wurde ein Pentadien-1,3 und Cyclopentadien enthaltender Strom abgezogen, der in der Destillationskolonne 4 in einen am Kopf über Leitung 5 abgezogenen Isoprenstrom und einen am Sumpf über Leitung 6 abgezogenen Pentadien-1,3 und Cyclopentadien enthaltenden Strom aufgetrennt wurde, der auch dampfdruckmäßig mitgenommenes selektives Lösungsmittel enthielt. Das Sumpfprodukt der Destillation wurde mit dem Sumpfprodukt der extraktiven Destillation vereinigt, indem es der extraktiven Destillation am Sumpf zugeführt wurde. Am Sumpf der extraktiven Destillation wurde über Leitung 7 ein Pentadien-1,3 und Cyclopentadien enthaltender Strom, der ca. 95 Gew.% selektives Lösungsmittel enthielt, abgezogen und im Hydrierreaktor 8, dem über Leitung 9 Wasserstoff zugeführt wurde, an einem Palladium enthaltenden Aluminiumoxidträgerkatalysator hydriert, wobei Pentadien-1,3 zu Pentenen und Pentan und Cyclopentadien zu Cyclopenten und Cyclopentan hydriert wurde. Im Hydrierreaktor wurde eine Temperatur von 130 °C und ein Druck von 30 bar aufrechterhalten. Der aus dem Hydrierreaktor abgezogene hydrierte Strom wurde über Leitung 10 dem Kopf der extraktiven Destillation zugeführt. Am Kopf der extraktiven Destillation wurde über Leitung 11 ein Pentane, Pentene, Cyclopentan und Cyclopenten enthaltendes Destillat abgezogen. Die über Leitung 7, Hydrierreaktor und Leitung 10 umlaufende Menge an selektivem Lösungsmittel wurde so eingestellt, daß die im Zulauf 6 zur extraktiven Destillation enthaltenen Pentadien-1,3 und Cyclopentadien bei einer Sumpftemperatur in der extraktiven Destillation von 110 bis 130 °C gelöst blieben.

In einem Vergleichsversuch wurde wie im vorstehenden Beispiel verfahren, wobei jedoch die Hydrierung durch Kurzschließen der Leitungen 7 und 10 umfahren wurde. Dabei reicherten sich Pentadien-1,3 und Cyclopentadien in der extraktiven Destillation stark an. Um daher Pentadien-1,3 und Cyclopentadien aus dem Sumpf der extraktiven Destillation 1 (nach Öffnung von Leitung 12) auszuschleusen, mußte die Sumpftemperatur in der extraktiven Destillation 1 stark angehoben werden, um eine ausreichende Ausstrippung der Pentadiene zu erreichen. Und zwar mußte die Sumpftemperatur in 1 bei 1,5 bar Arbeitsdruck bei Verwendung von wasserfreiem NMP als selektives Lösungsmittel auf über 200 °C und bei Verwendung von wasserhaltigem NMP (8 Gew.% $H_2O$) auf ca. 150 °C angehoben werden. Hohe Temperaturen begünstigen jedoch Dimerisation und Polymerisation der Diene. Durch eine Absenkung jedoch Dimerisation und Polymerisation der Diene. Durch eine Absenkung des Arbeitsdruckes in den Vakuumbereich konnte zwar die Sumpftemperatur gesenkt werden. Dies machte jedoch einerseits die Anwendung eines Kompressors oder von Kältemaschinen erforderlich, andererseits wurde durch die Verwendung dieser Apparate der Sauerstoffeintrag begünstigt. Sauerstoff ist jedoch ein wirksamer Initiator von überaus störenden Polymerisationen in Isoprenanlagen.

**Patentansprüche**

1. Verfahren zur Gewinnung von Isopren aus einem Isopren, Pentadien-1,3 und Cyclopentadien enthaltenden $C_5$-Kohlenwasserstoffgemisch durch Trennung des $C_5$-Kohlenwasserstoffgemischs durch Flüssig-Flüssig-Extraktion oder extraktive Destillation mit Hilfe eines selektiven Lösungsmittels in Kombination mit einer zur Flüssig-Flüssig-Extraktion oder extraktiven Destillation vorgeschalteten oder nachgeschalteten Destillation, dadurch gekennzeichnet, daß man in der vorgeschalteten oder nachgeschalteten Destillation einen Pentadien-1,3 und Cyclopentadien enthaltenden Strom abtrennt, diesen Strom katalytisch hydriert und den hydrierten Strom dem Oberteil der Flüssig-Flüssig-Extraktion oder der extraktiven Destillation zuführt.

2. Verfahren nach Anspruch 1, dadurch gekennzeichnet, daß man das Ausgangs-$C_5$-Kohlenwasserstoffgemisch zunächst in einer Destillationszone destilliert, wobei am Kopf der Destillationszone ein Isopren enthaltender Strom und am Sumpf der Destillationszone ein Pentadien-1,3 und Cyclopentadien enthaltender Strom abgezogen werden, den Pentadien-1,3 und Cyclopentadien enthaltenden Strom in einer Hydrierzone katalytisch hydriert, den hydrierten Strom dem Oberteil der Flüssig-Flüssig-Extraktion oder extraktiven Destillation zuführt, den am Kopf der Destillationszone abgezogenen, Isopren enthaltenden Strom dem mittleren Teil der Flüssig-Flüssig-Extraktion oder extraktiven Destillation zuführt und die der Flüssig-Flüssig-Extraktion oder extraktiven Destillation zugeführten $C_5$-Kohlenwasserstoffe in ein die im selektiven Lösungsmittel weniger als Isopren löslichen Kohlenwasserstoffe enthaltendes Destillat, einen Strom aus Isopren und einen die im selektiven Lösungsmittel löslicheren Kohlenwasserstoffe als Isopren enthaltenden Strom auftrennt.

3. Verfahren nach Anspruch 1, dadurch gekennzeichnet, daß man das Ausgangs-$C_5$-Kohlenwasserstoffgemisch zunächst durch Flüssig-Flüssig-Extraktion oder extraktive Destillation in ein die im selektiven Lösungsmittel weniger als Isopren löslichen Kohlenwasserstoffe enthaltendes Destillat, einen Isopren, Pentadien-1,3 und Cyclopentadien enthaltenden Strom und einen die im selektiven Lösungsmittel löslicheren Kohlenwasserstoffe enthaltenden Strom auftrennt, den Isopren, Pentadien-1,3 und Cyclopentadien enthaltenden Strom in einer Destillationszone auftrennt, wobei am Kopf der Destillationszone ein Strom aus Isopren und am Sumpf der Destillationszone ein Pentadien-1,3 und Cyclopentadien enthaltender Strom abgezogen werden, den Pentadien-1,3 und Cyclopentadien enthaltenden Strom in einer Hydrierzone katalytisch hydriert und den hydrierten Strom dem Oberteil der Flüssig-Flüssig-Extraktion oder extraktiven Destillation zuführt.

4. Verfahren nach Anspruch 1 bis 3, dadurch gekennzeichnet, daß man den Pentadien-1,3 und Cyclopentadien enthaltenden Strom mit selektivem Lösungsmittel verdünnt und danach katalytisch hydriert und den hydrierten mit selektivem Lösungsmittel verdünnten Strom dem Oberteil der Flüssig-Flüssig-Extraktion oder extraktiven Destillation zuführt.

5. Verfahren nach Anspruch 3, dadurch gekennzeichnet, daß man den am Sumpf der Destillationszone erhaltenen, Pentadien-1,3 und Cyclopentadien enthaltenden Strom dem unteren Teil des Ausgaserteils der Flüssig-Flüssig-Extraktion oder extraktiven Destillation zuführt, aus dem Ausgaserteil teilweise ausgegastes, Pentadien-1,3 und Cyclopentadien enthaltendes selektives Lösungsmittel abzieht und den erhaltenen Pentadien-1,3 und Cyclopentadien enthaltenden Lösungsmittelstrom anschließend katalytisch hydriert.

6. Verfahren nach Anspruch 3, dadurch gekennzeichnet, daß man den am Sumpf der Destillationszone erhaltenen, Pentadien-1,3 und Cyclopentadien enthaltenden Strom dem aus dem Ausgaserteil der Flüssig-Flüssig-Extraktion oder extraktiven Destillation abgezogenen ganz oder teilweise ausgegasten selektiven Lösungsmittel zuführt und den erhaltenen, Pentadien-1,3 und Cyclopentadien enthaltenden Lösungsmittelstrom anschließend katalytisch hydriert.

7. Verfahren nach Anspruch 6, dadurch gekennzeichnet, daß man den am Sumpf der Destillationszone erhaltenen, Pentadien-1,3 und Cyclopentadien enthaltenden Strom teilweise ausgegastem selektivem Lösungsmittel zuführt.

8. Verfahren nach Anspruch 1 bis 7, dadurch gekennzeichnet, daß die Hydrierwärme für die Trennung des $C_5$-Kohlenwasserstoffgemischs in der Flüssig-Flüssig-Extraktion oder extraktiven Destillation verwendet wird.

## Claims

1. A process for recovering isoprene from a $C_5$-hydrocarbon mixture containing isoprene, penta-1,3-diene and cyclopentadiene by separating the said mixture by liquid-liquid extraction or extractive distillation with the aid of a selective solvent in combination with a distillation upstream or downstream of the liquid-liquid extraction or the extractive distillation, wherein a stream containing penta-1,3-diene and cyclopentadiene is separated off in the upstream or downstream distillation and is catalytically hydrogenated, and the hydrogenated stream is fed to the upper section of the liquid-liquid extraction or extractive distillation unit.

2. A process as claimed in claim 1, wherein the starting $C_5$-hydrocarbon mixture is first distilled in a distillation zone, an isoprene-containing stream being taken off at the top of this zone and a stream containing penta-1,3-diene and cyclopentadiene being removed at the bottom of this zone, the stream containing penta-1,3-diene and cyclopentadiene is catalytically hydrogenated in a hydrogenation zone, the hydrogenated stream is fed to the upper section of the liquid-liquid extraction or extractive distillation unit, the isoprene-containing stream taken off at the top of the distillation zone is fed to the middle section of the liquid-liquid extraction or extractive distillation unit, and the $C_5$-hydrocarbons fed to the said extraction or said distillation are separated into a distillate which contains those hydrocarbons which are less soluble in the selective solvent than is isoprene, a stream consisting of isoprene, and a stream which contains those hydrocarbons which are more soluble in the selective solvent than is isoprene.

3. A process as claimed in claim 1, wherein the starting $C_5$-hydrocarbon mixture is first separated, by liquid-liquid extraction or extractive distillation, into a distillate containing those hydrocarbons which are less soluble in the selective solvent than is isoprene, a stream containing isoprene, penta-1,3-diene and cyclopentadiene, and a stream containing those hydrocarbons which are more soluble in the selective solvent, the stream containing isoprene, penta-1,3-diene and cyclopentadiene is separated in a distillation zone, a stream consisting of isoprene being taken off at the top of the said zone and a stream containing penta-1,3-diene and cyclopentadiene being removed at the bottom of the said zone, the stream containing penta-1,3-diene and cyclopentadiene is catalytically hydrogenated in a hydrogenation zone, and the hydrogenated stream is fed to the upper section of the liquid-liquid extraction or extractive distillation unit.

4. A process as claimed in claims 1 to 3, wherein the stream containing penta-1,3-diene and cyclopentadiene is diluted with the selective solvent and then hydrogenated catalytically, and the hydrogenated stream diluted with the selective solvent is fed to the upper section of the liquid-liquid extraction or extractive distillation unit.

5. A process as claimed in claim 3, wherein the stream which contains penta-1,3-diene and cyclopentadiene and is obtained at the bottom of the distillation zone is fed to the lower part of the devolatilization section of the liquid-liquid extraction or extractive distillation unit, partially devolatilized selective containing penta-1,3-diene and cyclopentadiene is taken off from the devolatilization section, and the resulting solvent stream containing penta-1,3-diene and cyclopentadiene is then catalytically hydrogenated.

6. A process as claimed in claim 3, wherein the stream which contains penta-1,3-diene and cyclopentadiene and is obtained at the bottom of the distillation zone is fed into the partially or completely devolatilized selective solvent removed from the devolatilization section of the liquid-liquid extraction or extractive distillation unit, and the resulting solvent stream containing penta-1,3-diene and cyclopentadiene is then catalytically hydrogenated.

7. A process as claimed in claim 6, wherein the stream which contains penta-1,3-diene and cyclopentadiene and is obtained at the bottom of the distillation zone is fed into partially devolatilized selective solvent.

8. A process as claimed in claims 1 to 7, wherein the heat of hydrogenation is used for the separation of the $C_5$-hydrocarbon mixture in the liquid-liquid extraction or extractive distillation.

## Revendications

1. Procédé d'obtention d'isoprène à partir d'un mélange d'hydrocarbures en $C_5$, contenant isoprène, pentadiène-1,3 et cyclopentadiène, par séparation du mélange d'hydrocarbures en $C_5$, au moyen d'une extraction liquide-liquide ou distillation extractive, à l'aide d'un solvant sélectif en combinaison avec une distillation précédant ou suivant l'extraction liquide-liquide ou la distillation extractive, caractérisé par le fait que dans la distillation précédente ou suivante, on sépare un courant contenant du pentadiène-1,3 et du cyclopentadiène, on hydrogène catalytiquement ce courant et on conduit le courant hydrogéné à la partie supérieure de l'extraction liquide-liquide ou de la distillation extractive.

2. Procédé selon la revendication 1, caractérisé par le fait que l'on distille le mélange d'hydrocarbures en $C_5$ de départ, tout d'abord, dans une zone de distillation, un courant contenant de l'isoprène étant prélevé en tête de la zone de distillation et un courant contenant du pentadiène-1,3 et du cyclopentadiène étant prélevé au fond de la zone de distillation, on hydrogène catalytiquement le courant contenant le pentadiène-1,3 et le cyclopentadiène dans une zone d'hydrogénation, on amène le courant hydrogéné à la partie supérieure de l'extraction liquide-liquide ou distillation extractive, on amène le courant contenant l'isoprène, et prélevé en tête de la zone de distillation, à la partie médiane de l'extraction liquide-liquide ou distillation extractive, et on sépare les hydrocarbures en $C_5$, amenés à l'extraction liquide-liquide ou distillation extractive, dans un distillat contenant les hydrocarbures moins solubles dans du solvant sélectif que l'isoprène, un courant d'isoprène et un courant contenant des hydrocarbures plus solubles que l'isoprène dans du solvant sélectif.

3. Procédé selon la revendication 1, caractérisé par le fait que l'on sépare le mélange d'hydrocarbures en $C_5$ de départ, d'abord, par extraction liquide-liquide ou distillation extractive, dans un distillat contenant les hydrocarbures moins solubles dans du solvant sélectif que l'isoprène, un courant contenant isoprène, pentadiène-1,3 et cyclopentadiène, et un courant contenant des hydrocarbures plus solubles que l'isoprène dans du solvant sélectif, on sépare, dans une zone de distillation, le courant contenant isoprène, pentadiène-1,3 et cyclopentadiène, un courant contenant de l'isoprène étant prélevé en tête de la zone de distillation et un courant contenant du pentadiène-1,3 et du cyclopentadiène étant prélevé au fond de la zone de distillation, on hydrogène catalytiquement le courant contenant le pentadiène-1,3 et le cyclopentadiène dans une zone d'hydrogénation, et on amène le courant hydrogéné à la partie supérieure de l'extraction liquide-liquide ou distillation extractive.

4. Procédé selon les revendications 1 à 3, caractérisé par le fait que l'on dilue le courant contenant pentadiène-1,3 et cyclopentadiène avec du solvant sélectif puis on hydrogène catalytiquement et on amène le courant dilué avec le solvant sélectif et hydrogéné à la partie supérieure de l'extraction liquide-liquide ou distillation extractive.

5. Procédé selon la revendication 3, caractérisé par le fait qu'on amène le courant contenant pentadiène-1,3 et cyclopentadiène, obtenu au fond de la zone de distillation, à la partie inférieure de la partie dégazage de l'extraction liquide-liquide ou distillation extractive, on prélève de la partie gazéification du solvant sélectif, contenant pentadiène-1,3 et cyclopentadiène, et partiellement dégazé, et on hydrogène enfin catalytiquement le courant de solvant contenant pentadiène-1,3 et cyclopentadiène.

6. Procédé selon la revendication 3, caractérisé par le fait que l'on amène le courant contenant pentadiène-1,3 et cyclopentadiène, obtenu au fond de la zone de distillation, au solvant sélectif totalement ou partiellement dégazé, et prélevé à la partie dégazage de l'extraction liquide-liquide ou distillation extractive, et on hydrogène enfin catalytiquement le courant de solvant obtenu, contenant pentadiène-1,3 et cyclopentadiène.

7. Procédé selon la revendication 6, caractérisé par le fait que l'on amène le courant, contenant pentadiène-1,3 et cyclopentadiène, obtenu au fond de la zone de distillation, au solvant sélectif partiellement dégazé.

8. Procédé selon les revendications 1 à 7, caractérisé par le fait que l'on utilise la chaleur d'hydrogénation pour la séparation du mélange d'hydrocarbures en $C_5$ dans l'extraction liquide-liquide ou distillation extractive.